(19) Europäisches Patentamt
European Patent Office
Office européen des brevets

(11) Veröffentlichungsnummer: **0 150 758**
**A2**

# (12) EUROPÄISCHE PATENTANMELDUNG

(21) Anmeldenummer: **85100360.8**

(22) Anmeldetag: **16.01.85**

(51) Int. Cl.⁴: **C 12 P 19/06**, C 08 B 37/00

(30) Priorität: **27.01.84 DE 3402757**

(43) Veröffentlichungstag der Anmeldung: **07.08.85**
**Patentblatt 85/32**

(84) Benannte Vertragsstaaten: **DE FR GB IT**

(71) Anmelder: **HOECHST AKTIENGESELLSCHAFT, Postfach 80 03 20, D-6230 Frankfurt am Main 80 (DE)**

(72) Erfinder: **Fischer, Edgar. Dr., Assmannshäuser Weg 8, D-6000 Frankfurt am Main 71 (DE)**
Erfinder: **Schlingmann, Merten. Dr., Schneidhainer Strasse 32a, D-6240 Königstein/Taunus (DE)**
Erfinder: **Dürsch. Walter. Dr., In der Braubach 4, D-6240 Königstein/Taunus (DE)**
Erfinder: **von Halasz, Sigmar-Peter Dr., Die Ritterwiesen 1c, D-6237 Liederbach (DE)**

## (54) Abtrennung carboxygruppenhaltiger Polysaccharide aus ihren wässrigen Lösungen.

(57) Quaternäre Ammoniumsalze, die eine alkalisch spaltbare langkettige aliphatische Gruppe besitzen, eignen sich zur Fällung carboxygruppenhaltiger Polysaccharide. Aus den so erhaltenen Addukten wird durch Einwirkung von Alkali das Polysaccharid wieder freigesetzt, wobei auch die Aminverbindung gespalten wird. Bei der Herstellung viskoser wässriger Zubereitungen brauchen die Spaltprodukte des Amins nicht abgetrennt zu werden.

EP 0 150 758 A2

HOECHST AKTIENGESELLSCHAFT HOE 84/F 019 Dr.KL/mü

Abtrennung carboxygruppenhaltiger Polysaccharide aus ihren wäßrigen Lösungen

Die Erfindung betrifft ein Verfahren zur Fällung carboxygruppenhaltiger Polysaccharide aus ihren wäßrigen Lösungen, insbesondere aus Fermentationslösungen und Abwässern, mit quaternären Ammoniumsalzen, die so erhaltenen Addukte und ihre Verwendung.

Aus der US-Patentschrift 3 119 182 ist es bekannt, carboxygruppenhaltige mikrobielle Polysaccharide wie Xanthan aus ihren Fermenationslösungen als Komplex mit einem quaternären Amin zu fällen. Als quaternäre Amine kommen Salze vom Typ des Cetyltrimethylammoniumchlorids in Betracht. Zur Fällung müssen die Fermentationslösungen entweder mindestens auf das 5-fache verdünnt werden, oder aber Alkalimetallhalide zugesetzt werden bzw. bei geringerer Verdünnungsrate eine Kombination von Verdünnung und Salzzusatz erfolgen. Die Aufspaltung der gefällten Addukte erfolgt durch wiederholtes Waschen mit alkalimetallchloridhaltigem Methanol. Die methanolischen Waschlösungen, die das Fällungsmittel enthalten, werden konzentriert und in den Prozeß zurückgeführt.

Es wurde nun gefunden, daß beim Einsatz solcher quaternärer Ammoniumsalze, die eine alkalisch spaltbare langkettige aliphatische Gruppe besitzen, zur Fällung der carboxygruppenhaltigen Polysaccharide weder eine Verdünnung noch ein Alkalimetallchloridzusatz nötig sind. Die so erhaltenen Niederschläge können mit alkoholischen oder wäßrigen Alkalien gespalten werden, das heißt, es ist auch im Spaltungsschritt kein Salzzusatz erforderlich, was die Abwasseraufarbeitung erheblich erleichtert. Bei der alkalischen Freisetzung des Polysaccharids aus seinem Addukt wird das Fällungsmittel gespalten, wobei Spaltstücke erhalten werden, die den Einsatz des Polysaccharids als Verdickungsmittel nicht stören. Eine Abtrennung dieser Spaltprodukte ist somit nicht erforderlich.

Die Erfindung betrifft somit ein Verfahren zur Abtrennung von carboxygruppenhaltigen Polysacchariden aus ihren wäßrigen Lösungen durch Fällung mit quaternären Ammoniumsalzen, die einen langkettigen aliphatischen Rest besitzen, das dadurch gekennzeichnet ist, daß ein quaternäres Ammoniumsalz der allgemeinen Formel I

$$[R^1-(CO)_m-(O-CHR^2-CHR^3)_n-O-COCHR^4-\overset{+}{N}(R^5)_3]X^- \quad (I)$$

eingesetzt wird, in der

$R^1$ einen geradkettigen oder verzweigten aliphatischen, araliphatischen oder aliphatisch-substitutierten aromatischen Rest mit 8 bis 22 Kohlenstoffatomen, vorzugsweise einen aliphatischen oder aliphatisch-substituierten aromatischen Rest mit 10 bis 18 Kohlenstoffatomen,

$R^2$, $R^3$ und $R^4$, unabhängig voneinander, Wasserstoff oder Methyl, jeweils vorzugsweise Wasserstoff, insbesondere alle Wasserstoff,

$R^5$ Alkyl mit 1 bis 3 Kohlenstoffatomen, vorzugsweise Methyl,

$X^-$ ein Anion, vorzugsweise ein Halogenid, insbesondere Chlorid,

m 0 oder 1, vorzugsweise 0, und

n eine Zahl von 0 bis 30, vorzugsweise 0 bis 10

bedeuten.

Die Verbindungen der Formel I sind bekannt, beispielsweise aus den deutschen Auslegeschriften 1 045 101 und 1 176 361 und sind erhältlich durch Alkylieren von tertiären Aminen der allgemeinen Formel II

$$N(R^5)_3 \quad (II) ,$$

mit Alkylierungsmitteln der Formel III

$$R^1-(CO)_m-(O-CHR^2-CHR^3)_n-O-CO-CHR^4-X \qquad (III)$$

wobei die Variablen in diesen beiden Formeln die für Formel I genannte Bedeutung haben. Diese Verbindungen werden im alkalischen Medium an der Estergruppe gespalten und zerfallen in den entsprechenden Alkohol und Betain.

Die bei dem erfindungsgemäßen Verfahren erhaltenen Addukte aus Polysaccharid und dem Kation des Salzes der Formel I sind neu und ebenfalls Gegenstand der Erfindung. Als Polysaccharide kommen solche in Betracht, die polyanionisch sind, das heißt neben den Hydroxygruppen noch Carboxyreste enthalten, beispielsweise die in der vorstehend genannten US-Patentschrift 3 119 812 genannten mikrobiell erhaltenen Produkte, insbesondere das Xanthan. Ferner sind beispielsweise Carboxymethyl- bzw. 2-Carboxyethyl-cellulose oder -stärke geeignet.

Die zu einer quantitativen Fällung der Polysaccharide erforderliche Menge an quaternärem Ammoniumsalz kann von der stöchiometrischen Menge abweichen und ist in gewissen Grenzen vom Polysaccharid und von der Natur des Salzes abhängig. Die zweckmäßigste Menge kann an Hand von einfachen Vorversuchen ermittelt werden. Generell wird die Abtrennbarkeit mit steigender Hydrophobie des langkettigen Restes verbessert. Wenn also die Gruppe $R^1$ wenig hydrophob ist, darf die Polyalkoxykette nicht stark hydrophil sein, das heißt n kann gegen 0 gehen, falls $R^2$ und $R^3$ Wasserstoff sind. Ist $R^1$ stark hydrophob, also beispielsweise ein Alkylrest mit 18 und mehr Kohlenstoffatomen, so kann n einen Wert an der Obergrenze des definierten Bereichs annehmen, auch wenn $R^3$ und $R^4$ für Wasserstoff stehen.

Die Fällung der mikrobiellen Polysaccharide aus ihren bei der Fermentation anfallenden, ca. 1,5 bis 2 Gew.-% Polysaccharid enthaltenden Lösungen erfolgt in an sich

bekannnter Weise durch Zugabe von Lösungen der quaternären Ammoniumsalze der Formel I, zweckmäßig unter intensiver mechanischer Beanspruchung mit Hilfe von Apparaturen, die eine Rühr-, Schneid-, Mahl- oder Scherwirkung auf das ausgefällte Produkt ausüben können.

Zur Freisetzung der Polysaccharide aus den Addukten werden diese zweckmäßig in der durch Abfiltrieren, Absaugen, Abdrücken oder Abzentrifugieren erhaltenen feuchten Form, also als Paste oder Preßkuchen, eingesetzt. Es entfällt so das Trocknen der Addukte, das nicht nur beachtliche Energiemengen erfordert, sondern auch das Löslichkeits- und Quellvermögen beeinträchtigen kann. Die Feststoffgehalte der Pasten bzw. Preßkuchen liegen im allgemeinen bei etwa 4 bis 40 Gew.-%, bevorzugt bei 6 bis 30 Gew.-%.

Erfindungsgemäß werden die Zwischenprodukte zur Herstellung der freien Polysaccharide verwendet. Die Spaltung der Addukte erfolgt hierbei mit Alkalien in Wasser und/oder einem niederen Alkanol. Als alkalische Mittel kommen Ammoniak, leicht flüchtige Amine mit einem Siedepunkt bei Normaldruck von unter etwa 150°C, beispielsweise Triethylamin, sowie basische Derivate der Alkali- und Erdalkalimetalle, wie beispielsweise die Hydroxide, Alkoholate und Carbonate des Natriums, Kaliums und Lithiums und die Hydroxide des Magnesiums, Calciums und Bariums in Betracht. Ammoniak ist wegen seiner Flüchtigkeit besonders geeignet und wird zweckmäßig in Form seiner wäßrigen Lösungen eingesetzt.

Bei dieser alkalischen Spaltung fallen die Polymere in Form ihrer Carboxylat-Salze an, die jedoch hier und im folgenden - im Gegensatz zum Amin-Addukt - als "freie" Polysaccharide bezeichnet werden, auch wenn sie nicht in die freie Säureform überführt sind.

Für einige Einsatzzwecke der Polysaccharide ist es unerwünscht, wenn bei ihrem Einsatz in wäßrigen Systemen zu hohe pH-Werte von beispielsweise über 10 vorliegen. Es sollten deshalb zu hohe molare Überschüsse an alkalischem Mittel, bezogen auf das Polysaccharid und das im Addukt gebundene Amin, von vornherein vermieden werden. Hierdurch werden auch hydrolytische Abbaureaktionen unterdrückt. Andererseits sind gewisse Mindestmengen an alkalischem Mittel notwendig, um die im Polysaccharid-Amin-Addukt salzartig gebundenen Amine zunächst freizusetzen und anschließend noch zu spalten. Die Mindestmengen an alkalischem Mittel liegen bei etwa 0,7 bis 1 Val pro Val Carboxygruppen. Zweckmäßig wird das 1,2- bis 8-fache, bevorzugt das 1,5- bis 4-fache, der Mindestmenge an alkalischem Mittel eingesetzt. Im allgemeinen setzt man etwa 1 bis 10, vorteilhaft 2 bis 8, Val Alkali pro 1000 g 100 %igem Polymer ein. Die hydrolytische Spaltung der Amine erfolgt unter diesen Bedingungen bereits langsam bei Raumtemperatur und ist je nach Alkalikonzentration nach 1 bis 60 Minuten beendet. Ein Erhitzen auf etwa 30 bis 60°C beschleunigt die Spaltung.

Die Spaltung kann auf verschiedene Weise erfolgen:

Wenn das Addukt isoliert und getrocknet wurde, wird unter Zugabe der berechneten Menge an alkalischem Mittel die Spaltung im wäßrigen, wäßrig-alkoholischen oder alkoholischen Medium durchgeführt. Im wasserfreien alkoholischen Medium werden alkohollösliche alkalische Mittel eingesetzt, beispielsweise Ammoniak, Alkali- oder Erdalkalihydroxide bzw. -alkoholate, die sich vorzugsweise vom gleichen Alkohol ableiten, der als Reaktionsmedium dient.

Bei der Spaltung wasserhaltiger Pasten oder Preßkuchen erfolgt die Spaltung im wäßrigen oder wäßrig-alkoholischen Medium, wobei sich die Menge des alkalischen Mittels am

Trockengehalt des Addukts und am Carboxygruppen-Äquivalent des Polysaccharids orientiert.

In einer vorteilhaften Ausführungsform der Erfindung wird das alkalische Mittel zu der Fällungssuspension gegeben, wobei also nicht die Addukte, sondern gleich die freien Polysaccharide isoliert werden.

Die freigesetzten Polysaccharide können nach der Isolierung durch Filtrieren oder Zentrifugieren getrocknet oder aber unmittelbar in feuchter Form als wäßrige Pasten oder Preßkuchen eingesetzt werden. Für die Verwendung als Verdickungsmittel stört im allgemeinen ein Gehalt an den Spaltprodukten der Aminkomponente (langkettiger Alkohol und Betain) nicht. Sollen diese Spaltprodukte abgetrennt werden, so kann dies durch Extraktion mit organischen wassermischbaren Lösemitteln erfolgen. Zweckmäßig werden hierzu die wäßrigen Konzentrate, also Preßkuchen oder Pasten, mit der 2- bis 10-fachen, bevorzugt 3- bis 6-fachen, Menge des organischen wassermischbaren Lösemittels behandelt. Einwertige niedere Alkohole mit bis zu 3 Kohlenstoffatomen sind bevorzugt, insbesondere Isopropanol. Die in diesen Lösemitteln unlöslichen Polysaccharide fallen als leicht abscheidbare Niederschläge an und können einfach physikalisch abgetrennt werden, z. B. durch Absaugen oder Abzentrifugieren.

Die isolierten Polysaccharide können noch geringe Mengen an Alkaliionen enthalten, die durch mehrmaliges Nachwaschen mit Alkanolen, bevorzugt Methanol, größtenteils entfernt werden können. Sofern gewünscht, können Reste der Alkaliionen durch Zusatz einer geringen Menge einer geeigneten Säure zum letzten Wasch-Alkanol beseitigt bzw. in die entsprechenden, beim praktischen Einsatz vernachlässigbaren Salze übergeführt werden. Hierfür geeignete Säuren sind Mineralsäuren wie Salzsäure, Schwefelsäure oder Phosphorsäure, vorzugsweise jedoch

organische Säuren wie Ameisensäure, Essigsäure, Milchsäure, Weinsäure, Äpfelsäure oder Zitronensäure. Die so erhaltenen und gegebenenfalls getrockneten Endprodukte lassen sich in wäßrigen Systemen einsetzen, in denen ein pH-Wert im Bereich von etwa 5 bis 7 erforderlich ist.

Auch wenn die Spaltprodukte der Aminkomponente nicht abgetrennt werden, werden Produkte erhalten, deren Viskositätseigenschaften in wäßrigen Systemen nicht beeinträchtigt sind. Die mit solchen Produkten erhaltenen wäßrigen Systeme zeichnen sich also durch gute Viskositätseigenschaften aus. Die erfindungsgemäß erhaltenen Polysaccharide können somit weitgehend unabhängig von ihrem Reinheitsgrad als Verdickungsmittel für wäßrige Systeme dienen.

Die Erfindung wird durch die folgenden Beispiele näher erläutert. Prozentangaben und Relationen beziehen sich hierbei auf das Gewicht, sofern nichts anderes angegeben ist.

Die Viskositäten wurden wie folgt bestimmt:

Es wurden Lösungen bereitet, die jeweils 0,1 % (1000 ppm) Xanthan enthielten, wobei als Lösemittel voll entsalztes elektrolytfreies Wasser (im folgenden E-Wasser) bzw. eine Standardsalzlösung diente, die pro Liter 130 g Natriumchlorid und 10 g Calciumchlorid enthielt. Die mit E-Wasser erhaltenen Viskositätswerte werden als $\eta_E$ bezeichnet, entsprechend die mit der Salzlösung erhaltenen Werte als $\eta_S$. Die Messung erfolgte mit dem Viskosimeter $^R$ROTOVISKO RV 100/CV 100 der Fa. Haake, Berlin, bei 22°C und einem Schergefälle von D= 10,0 $s^{-1}$. Die gefundenen Viskositäten sind in mPa·s angegeben.

Unter diesen Bedingungen wurden die in der folgenden Tabelle angegebenen Viskositäten erhalten.

Tabelle 1

| 0,1 % | $n_E$ | $n_S$ |
|---|---|---|
| Xanthan ([R] RHODOPOL 23)* | 31,0 | 41,0 |
| Carboxymethylcellulose | 1,39 | 1,45 |
| Carboxymethylstärke | 1,33 | 1,38 |
| - (= reines Lösemittel) | 1,15 | 1,24 |

* der Fa. Rhône-Poulenc

Beispiel 1

Zu 600 g einer 1,5 %igen Lösung von handelsüblichem Xanthan-Pulver (RHODOPOL 23) wurden unter intensivem Rühren mit Hilfe eines Mahlwerks (ULTRATURRAX) 16 g einer 25 %igen Lösung des Betain-n-dodecylester-chlorids der Formel

$$[H-(CH_2)_{12}-O-CO-CH_2-\overset{+}{N}(CH_3)_3]\ Cl^-$$

eingerührt. Nach 5 Minuten Rühren wurde das ausgefallene Xanthan-Amin-Addukt abgesaugt. Man erhielt 34,2 g eines feuchten Filterkuchens, der ca. 33 % Xanthan enthielt. Dieser wurde im Rotationsverdampfer bei 2 - 4 mbar 6 Stunden bei 40 - 45°C (Badtemperatur) vorsichtig getrocknet und anschließend gemahlen. Es wurden 12,1 g eines pulverförmigen Addukts erhalten, das etwa 70 % Xanthan enthielt.

Zur Herstellung der 0,1 %igen Lösungen wurden 1,43 g des pulverförmigen Addukts (= 1,0 g 100 %iges Xanthan) in E-Wasser und in Standardsalzlösung unter intensivem Rühren suspendiert. Nach 30 Minuten Rühren wurden jeweils 0,015 % Ammoniak in Form einer 2,5 %igen wäßrigen Lösung zugefügt und weitergerührt, bis das Addukt vollständig in Lösung gegangen war. Es wurden folgende Viskositätswerte gemessen:

$\eta_E$ 36,2 mPa·s
$\eta_S$ 38,0 mPa·s

Beispiel 2

Zu 34,2 g gemäß Beispiel 1 erhaltenem feuchtem Filterkuchen wurden 2,5 g 25 %iges wäßriges Ammoniak gegeben. Das Gemisch wurde 1 Stunde bei 40°C und 5 mbar im Rotationsverdampfer teilweise getrocknet. Es resultierte eine feuchte Paste, die neben geringen Mengen der Aminspaltprodukte (n-Dodecylalkohol und Betain) 21 % Xanthan enthielt.

Hieraus hergestellte, 0,1 % Xanthan enthaltende Lösungen zeigten folgende Viskositätswerte:

$\eta_E$= 48,0 mPa·s
$\eta_S$= 48,7 mPa·s

Beispiel 3

Zu 34,2 g gemäß Beispiel 1 erhaltenem feuchtem Filterkuchen wurden 182 g E-Wasser und 1,4 g 25 %iges wäßriges Ammoniak zugefügt und das Gemisch 30 Minuten bei 35°C gerührt. Man erhielt 217 g einer Paste, die ca. 5,4 % Xanthan enthielt.

0,1 % Xanthan enthaltende Lösungen zeigten folgende Viskositäten:

$\eta_E$= 37,6 mPa·s
$\eta_S$= 35,5 mPa·s

Beispiel 4

Zu 34,2 g gemäß Beispiel 1 erhaltenem feuchtem Filterkuchen wurden 200 g Isopropanol und 1,7 g 25 %iges wäßriges Ammoniak gegeben. Das Gemisch wurde mit Hilfe eines Schnellrührers (ULTRATURRAX) 5 Minuten gemahlen und der Xanthanniederschlag abgesaugt und abgepreßt. Der feuchte Filterkuchen (16,2 g) wurde im Ölpumpenvakuum bei 2 - 5 mbar getrocknet und anschließend gemahlen. Man erhielt 8,6 g eines dodecylalkohol- und betainfreien Xanthanpulvers.

Beispiele 5 - 14

Allgemeine Verfahrensweise:

Zu jeweils 600 g einer 1,5 %igen Lösung eines handelsüblichen Xanthanpulvers (RHODOPOL 23) wurden unter intensivem Rühren w g (s. Spalte 5 der Tabelle 2) einer x-%igen (Spalte 6) Lösung eines Betainesterchlorids der allgemeinen Formel Ia

$$[R^1-(CO)_m-(O-C_2H_4)_n-O-CO-CH_2-\overset{+}{N}(CH_3)_3]\ Cl^- \qquad Ia$$

gegeben. Die Bedeutungen von $R^1$, m und n sind in den Spalten 2, 3 und 4 der Tabelle II angegeben.

Nach 5 Minuten Rühren wurden die ausgefallenen Xanthan-Amin-Addukte abgesaugt. Die feuchten Filterrückstände wurden gewogen (y g in Spalte 7) und ihr %-Gehalt an Xanthan berechnet (z in Spalte 8).

Die Filterrückstände wurden in E-Wasser bzw. Standardsalzlösung derart suspendiert, daß jeweils 0,1 %ige Suspensionen, bezogen auf Xanthan, resultierten. Es wurden jeweils 0,015 % Ammoniak in Form einer 2,5 %igen wäßrigen Lösung zugefügt und bis zum Entstehen homogener Lösungen weitergerührt.

Die gefundenen Viskositätswerte sind in den Spalten 9 und 10 in mPa·s angegeben.

Ähnliche Viskositätsdaten wurden auch mit 0,1 %igen Xanthanlösungen erhalten, die aus Pasten mit einem Gehalt von 4 % Xanthan durch Verdünnen mit E-Wasser bzw. Standardsalzlösung hergestellt wurden, wobei diese Pasten ihrerseits durch Behandeln der Filterrückstände mit Standardsalzlösung gewonnen worden waren.

Tabelle 2

| 1<br>Beisp.-Nr. | 2<br>$R^1$ | 3<br>n | 4<br>m | 5<br>w | 6<br>x | 7<br>y | 8<br>z | 9<br>$n_E$ | 10<br>$n_S$ |
|---|---|---|---|---|---|---|---|---|---|
| 5 | $C_{10}H_{21}$ | 0 | 0 | 23 | 25 | 32,2 | 27,9 | 38,6 | 44,6 |
| 6 | $C_{12}H_{25}$ | 0 | 0 | 17 | 25 | 25,9 | 34,8 | 37,5 | 31,5 |
| 7 | i-$C_{13}H_{27}$ | 0 | 0 | 22 | 25 | 32,6 | 27,6 | 39,4 | |
| 8 | Tagfett-alkyl | 0 | 0 | 70 | 10 | 37,5 | 24,0 | 37,7 | 38,5 |
| 9 | Cocosfett-alkyl | 3 | 0 | 30 | 25 | 34,9 | 25,8 | 38,8 | 42,5 |
| 10 | Cocosfett-säurealkyl | 2 | 1 | 52 | 25 | 46,5 | 19,4 | 27,1 | 29,0 |
| 11 | $C_{18}H_{35}$ | 6 | 0 | 55 | 25 | 42,6 | 21,1 | 31,3 | 37,5 |
| 12 | $C_{18}H_{37}$ | 6 | 0 | 52 | 25 | 53,0 | 17,0 | 38,5 | 67,8 |
| 13 | 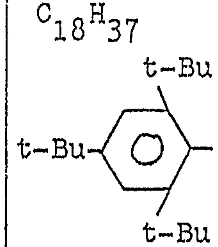 | 10 | 0 | 44 | 25 | 58,6 | 15,3 | 36,3 | 38,8 |
| 14 | $C_9H_{19}-C_6H_4$ | 8 | 0 | 45 | 25 | 74,0 | 12,2 | 41,7 | |

Beispiel 15

In einem Fermentationsansatz zur Herstellung von Xanthan wurden die Bakterienkultur vom Typ Xanthomonas campestris durch Erhitzen abgetötet und die Zellwände durch Zugabe von Alkalase aufgelöst. In 600 g des derart vorbereiteten Reaktionsgemischs, das ca. 1,9 % Xanthan enthielt, wurden mit Hilfe eines Schnellrührers 35 g einer 25 %igen Lösung des im Beispiel 1 eingesetzten Betain-n-dodecylesterchlorids eingerührt. Der sich abscheidende Addukt-Niederschlag wurde über eine Nutsche abgesaugt. Man erhielt 446 g einer schwach gelb gefärbten Addukt-Paste mit einem Xanthangehalt von 7,6 %.

Aus der so erhaltenen Paste wurden 0,1 %ige Suspensionen, bezogen auf Xanthan, in E-Wasser bzw. Standardsalzlösung hergestellt, unter Rühren 0,015 % Ammoniak zugegeben und weitergerührt, bis homogene Lösungen resultierten. Die Viskositätswerte waren wie folgt:

$\eta_E$= 39,7 mPa·s
$\eta_S$= 45,3 mPa·s

Beispiel 16

Zu 149 g der nach Beispiel 15 erhaltenen Adduktpaste wurden 5,0 g 25 %iges wäßriges Ammoniak zugegeben. Nach 20 Stunden Stehen bei Raumtemperatur wurde die Paste, in der neben Xanthan die Aminspaltprodukte vorlagen, auf einen Xanthangehalt von 0,1 % mit E-Wasser verdünnt. Die Viskosität der erhaltenen Lösung lag bei $\eta_E$= 32,3 mPa·s.

Nach dem Verdünnen der schwach ammoniakalischen Paste mit Standardsalzlösung auf 0,1 % ergab sich eine Viskosität $\eta_S$= 39,9 mPa·s.

Beispiel 17

Zu 877 g einer 1,5 %igen Lösung von Carboxymethylcellulose (CMC) in E-Wasser wurden mit Hilfe eines Schnellrührers (ULTRATURRAX) 68 g einer 25 %igen Lösung des im Beispiel 1 definierten Betain-n-dodecylchlorids eingerührt. Der Addukt-Niederschlag wurde abgesaugt, wobei 72,5 g Filterrückstand mit einem CMC-Gehalt von 17,2 % erhalten wurden. Hiervon wurden jeweils 0,1 %ige Suspensionen, bezogen auf CMC, in E-Wasser bzw. Standardsalzlösung hergestellt, pro 100 g Suspension 0,5 g 2,5 %iges wäßriges Ammoniak zugefügt und weitergerührt, bis das Addukt sich vollständig aufgelöst hatte. Viskositäten:

$n_E$= 6,80 mPa•s
$n_S$= 2,19 mPa•s

Beispiel 18

Es wurde verfahren wie bei Beispiel 17, jedoch 213 g einer 10 %igen Lösung des Betain-talgfettalkyl-esterchlorids der Formel

$$[\text{Talgfettalkyl-O-CO-CH}_2\text{-}\overset{+}{\text{N}}(\text{CH}_3)_3]^+ \text{Cl}^-$$

eingesetzt. Nach Absaugen wurden 177,4 g Addukt mit einem Gehalt von 7,0 % CMC erhalten.

Durch Suspendieren in E-Wasser bzw. Standardsalzlösung und Zugabe von jeweils 0,5 g 2,5 %igem wäßrigem Ammoniak pro 100 g Lösung erhielt man Lösungen mit folgenden Viskositäten:

$n_E$= 6,35 mPa•s
$n_S$= 3,64 mPa•s

Beispiel 19

Zu 877 g einer 1,5 %igen Lösung von Carboxymethylstärke (CMS) in E-Wasser wurden 70 g einer 25 %igen Lösung des im Beispiel 1 definierten Betain-n-dodecylesterchlorids eingerührt. Nach Absaugen wurden 44,5 g Addukt mit einem Gehalt von 28,1 % CMS erhalten. Nach Spaltung des Addukts durch Zugabe von je 0,5 g 2,5 %igem Ammoniak pro 100 g Lösung zeigten die 0,1 % CMS enthaltenden Lösungen die folgenden Viskositäten:

$\eta_E$= 2,25 mPa·s
$\eta_S$= 2,11 mPa·s

Beispiel 20

Zu 600 g einer 1,5 %igen Lösung von handelsüblichem Xanthan-Pulver (RHODOPOL 23) wurden unter intensivem Rühren 35 g einer 25 %igen Lösung eines Salzgemischs der Formel

$$[H-(CH_2)_a-O-CO-CH_2-\overset{+}{N}(CH_3)_3]Cl^-$$

in der a für 10 und 12 steht, eingerührt. Man erhielt nach dem Absaugen 104 g einer Xanthan-Amin-Addukt-Paste mit einem Xanthangehalt von 8,7 %. Hiervon wurden jeweils 0,1 % Xanthan enthaltende Suspensionen in E-Wasser und in Standardsalzlösung hergestellt und zu jeweils 100 g dieser Suspensionen entweder 0,5 g 2,5 %ige wäßrige Ammoniaklösung oder 1 g 1 N Natronlauge zugesetzt. Durch Rühren wurden homogene Lösungen erhalten, die folgende Viskositäten (in mPa·s) zeigten:

| Alkali | $\eta_E$ | $\eta_S$ |
|---|---|---|
| $NH_3$ | 45,2 | 27,8 |
| NaOH | 42,6 | 29,0 |

Patentansprüche:

1. Verfahren zur Abtrennung von carboxygruppenhaltigen Polysacchariden aus ihren wäßrigen Lösungen durch Fällung mit quaternären Ammoniumsalzen, die einen langkettigen aliphatischen Rest besitzen, dadurch gekennzeichnet, daß ein quaternäres Ammoniumsalz der allgemeinen Formel I

$$[R^1-(CO)_m-(O-CHR^2-CHR^3)_n-O-COCHR^4-\overset{+}{N}(R^5)_3]X^- \quad (I)$$

eingesetzt wird, in der

$R^1$ einen geradkettigen oder verzweigten aliphatischen, araliphatischen oder aliphatisch-substituierten aromatischen Rest mit 8 bis 22 Kohlenstoffatomen,

$R^2$, $R^3$ und $R^4$, unabhängig voneinander, Wasserstoff oder Methyl,

$R^5$ Alkyl mit 1 bis 3 Kohlenstoffatomen,

$X^-$ ein Anion,

m 0 oder 1 und

n eine Zahl von 0 bis 30

bedeuten.

2. Verfahren nach Anspruch 1, dadurch gekennzeichnet, daß ein quaternäres Ammoniumsalz der allgemeinen Formel I eingesetzt wird, in der $R^1$ einen geradkettigen oder verzweigten aliphatischen oder aliphatisch-substituierten aromatischen Rest mit 10 bis 18 Kohlenstoffatomen,

$R^2$, $R^3$ und $R^4$ Wasserstoff,

$R^5$ Methyl,

$X^-$ ein Halogenid,

m 0 und

n eine Zahl von 0 bis 10

bedeuten.

3. Quaternäre Ammoniumsalze der allgemeinen Formel I, in der $X^-$ das Anion eines carboxygruppenhaltigen Polysaccharids bedeutet und die übrigen Reste wie in Anspruch 1 definiert sind.

4. Verbindungen gemäß Anspruch 3, bei den $R^1$, $R^2$, $R^3$, $R^4$, $R^5$, m und n wie im Anspruch 2 definiert sind.

5. Verbindungen nach Anspruch 3 oder 4, dadurch gekennzeichnet, daß $X^-$ das Anion des Xanthans bedeutet.

6. Verwendung der Verbindungen nach Anspruch 3 bis 5 als Ausgangsmaterialien zur Freisetzung der darin enthaltenen carboxygruppenhaltigen Polysaccharide durch alkalische Spaltung.

Patentansprüche Österreich:

1. Verfahren zur Abtrennung von carboxygruppenhaltigen Polysacchariden aus ihren wäßrigen Lösungen durch Fällung mit quaternären Ammoniumsalzen, die einen langkettigen aliphatischen Rest besitzen, dadurch gekennzeichnet, daß ein quaternäres Ammoniumsalz der allgemeinen Formel I

$$[R^1-(CO)_m-(O-CHR^2-CHR^3)_n-O-COCHR^4-\overset{+}{N}(R^5)_3]X^- \quad (I)$$

eingesetzt wird, in der

$R^1$ einen geradkettigen oder verzweigten aliphatischen, araliphatischen oder aliphatisch-substituierten aromatischen Rest mit 8 bis 22 Kohlenstoffatomen,

$R^2$, $R^3$ und $R^4$, unabhängig voneinander, Wasserstoff oder Methyl,

$R^5$ Alkyl mit 1 bis 3 Kohlenstoffatomen,

$X^-$ ein Anion,

m 0 oder 1 und

n eine Zahl von 0 bis 30

bedeuten.

2. Verfahren nach Anspruch 1, dadurch gekennzeichnet, daß ein quaternäres Ammoniumsalz der allgemeinen Formel I eingesetzt wird, in der $R^1$ einen geradkettigen oder verzweigten aliphatischen oder aliphatisch-substituierten aromatischen Rest mit 10 bis 18 Kohlenstoffatomen,

$R^2$, $R^3$ und $R^4$ Wasserstoff,

$R^5$ Methyl,

$X^-$ ein Halogenid,

m 0 und

n eine Zahl von 0 bis 10

bedeuten.

3. Verwendung nach Anspruch 1 oder 2 erhältlichen Verbindungen als Ausgangsmaterialien zur Freisetzung der darin enthaltenen carboxygruppenhaltigen Polysaccharide durch alkalische Spaltung.